# EUROPEAN PATENT APPLICATION

(11) **EP 4 706 734 A2**
(43) Date of publication of application: **11.03.2026**
(21) Application number: 25223653.4
(22) Date of filing: 24.11.2021
(51) Int. Cl.: A61M 25/04

(54) **AN ANAL PROBE**

(30) Priority: 24.11.2020 DK PA202070779
(62) Divisional of application: 21823182.7
(71) Applicant: Coloplast A/S, 3050 Humlebaek (DK)
(72) Inventor: HICKMOTT, Richard Morgan, 3050 Humlebaek (DK); DOLRIIS, Jakob Duus, 3050 Humlebaek (DK)

(57) **Abstract**

An anal probe is provided. The probe has α retention part and α stem, where the retention part flares outward from its attachment to the stem and towards the insertion end. The retention part may be made of α soft resilient material.

## Description

The invention relates to an anal probe for use in an irrigation system.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figures 1 and 2 illustrate an anal probe, figure 1 illustrates a perspective view and figure 2 a cross-sectional view.
Figures 3 and 4 illustrate an anal probe, figure 3 illustrates a perspective view and figure 4 a cross-sectional view.
Figures 5 and 6 illustrate stems with different tip portions. Figures 5A, 5B illustrate open-ended tips and 6A, 6B illustrate closed tips with eyelets.
Figures 7A, 7B and 7C illustrate different positions of the tip with respect to the rim of the retention part.
Figures 8A and 8B illustrate a bowl-shaped retention part and the position of the tip with respect to the inner edge of the retention part.
Figures 9 and 10 illustrate a part of an anal probe in a compressed configuration and wrapped for storage.
Figure 11 illustrates a test set-up for testing vertical and radial pull out forces, respectively.
Figure 12 illustrates a cavity used in the test set-up in figure 11.
Figure 13 illustrates a test set-up used for testing drop out pressure and leakage.
Figures 14 and 15 illustrate a cavity in perspective view and cross-sectional view for use in the test set-up in figure 13.

### Detailed Description

Embodiments relate to an anal probe for use in an anal irrigation procedure, the anal probe comprising
- a stem having a distal end and a proximal tip portion, the stem comprising an internal lumen extending from the distal end to the proximal tip portion
- a retention part configured for being inserted into the rectum and having a distal portion and a proximal portion, the distal portion having a first radial extent which is smaller than a second radial extent proximally of the distal portion,
wherein the distal portion of the retention part comprises a central bore, which is fixedly attached, connected to or moulded to the stem at an attachment portion of the stem between the proximal portion of the stem and the distal end of the stem.

An anal probe as described above has the advantage of being very easy to use, since the retention part is built in and requires no manipulation from the user. The anal probe is self-sealing without the need to inflate a balloon.

In the following, whenever referring to a proximal end of an element of the invention, the referral is to the end adapted for insertion. Whenever referring to the distal end of an element, the referral is to the end opposite the insertion end. In other words, the proximal end is the end closest to the user, when the probe is to be inserted and the distal end is the opposite end - the end furthest away from the user when the probe is to be inserted.

The longitudinal direction is the direction from the distal to the proximal end. The transverse direction is the direction perpendicular to the longitudinal direction, which corresponds to the direction across the stem of the probe.

By radial extent is meant an extent in a transverse direction extending from one outer surface directly across in the transverse direction to the opposite outer surface.

An irrigation system typically comprises a reservoir or container for irrigation liquid, an anal probe and tubing connecting those two. The system may also include a pump for pumping the irrigation liquid into the intestines. When a self-sealing anal probe as described herein is used in an anal irrigation system, the system does not require an extra tube nor an extra pump for filling a balloon used as retention means. Therefore, the system can be simpler, when an anal probe as described herein is used.

The anal probe comprises a stem in the form of a tubular part extending from the distal end towards the proximal end. The tip portion is positioned in the proximal end of the stem. The probe may comprise a connector in the distal end of the stem.

The retention part is attached, connected or moulded to an attachment portion of the stem, which is between the proximal tip portion of the stem and the distal end of the stem. A central bore in the retention part is configured for being brought into contact with the attachment portion of the stem and the two parts are fixedly attached, connected or moulded together. The retention part increases in circumference from its distal portion towards the proximal portion of the retention part. The largest circumference of the retention part will be between approximately 15 mm and 220 mm corresponding to a radial extent across the retention part in the transverse direction between 5 mm and 70 mm. Larger retention parts having a radial extent up to 75 mm may be contemplated.

The tubular part of the stem has a diameter of 5-10 mm.

Distal portion of the retention part is defined as the portion of the retention part, which is attached to the stem at an attachment portion of the stem.

Proximal portion of the stem is defined as the portion of the stem, which extends proximally beyond the attachment.

The stem may be made of Polyethylene, Polypropylene, or a combination of a TPE with a harder material such as PE, PP or ABS. The stem may also be made of Polyurethane.

The retention part may be made of a soft resilient material.

The retention part may be made of foam, for example a polyurethane foam. The foam should be rather soft and compliant, but also function as self-sealing. An example of a suitable foam is RAKU-PUR^{®} 31-3173. Another example could be a foam as the one used in Biatain^{®} wound dressings marketed by Coloplast A/S.

A soft resilient material, such as foam, will have the beneficial effect of providing a self-sealing probe. This is because the resilient material will naturally conform to the bowel and when liquid is in the bowel on the proximal side of the retention part, this liquid will assist in pressing the flare of the retention part outwards such that the largest radial extent and circumference of the retention part may increase.

Furthermore, in examples where the retention part is made of foam, the foam may be saturated by the irrigation liquid and will (also) in this way increase the sealing towards the inside of the bowel.

The retention part may also be made of silicone.

The materials suitable for this purpose may have a hardness in the Shore 00 range of between 20 and 50.

If a sealing film is used as an impermeable layer, a thin Polyurethane film may be used. Examples of thicknesses are between 5 to 250 µm, such as 30 or 50 µm.

An examples of a complete probe is a probe where the stem, the retention part and the sealing film are all made of Polyurethane.

The retention part of the anal probe described herein may be compressed to a smaller diameter in a transverse direction, such that the diameter in a compressed configuration is around 20 % of the diameter in a use configuration. The compressed configuration may be supported by a film-element, which is wrapped around the retention part to keep it in that configuration. This makes the probe easier to insert. The use configuration, where the retention part is unfolded or uncompressed is required for providing a better sealing to the bowels during the irrigation procedure. The diameter, or the radial extent of the retention part in the compressed configuration may be 25 % or 30 % or lower such as 15 % or 10 % of the radial extent of the retention part in a use configuration.

In an embodiment, the proximal tip portion is an open tip. In other words, the inside lumen of the stem ends in an open end, so that irrigation liquid exits the proximal tip portion directly through the open end. The open end may simply be cut-off or it may be slightly rounded at the end of lumen so as to provide an atraumatic tip. During insertion of the probe, a user may inadvertently poke the probe around slightly; therefore, it is an advantage if the proximal end of the probe is atraumatic.

In an embodiment, the proximal tip portion is a closed tip. This means that the inside lumen of the stem is closed off in the proximal end and a sidewall of the proximal tip portion is provided with at least one eyelet for allowing irrigation liquid to exit the proximal tip portion. More than one eyelet may be used, such as two or three. The proximal closed end of the proximal tip portion may be rounded, or it may be flat or angled. If the closed end of the proximal tip portion is the proximal end of the anal probe, it may be an advantage that the closed end is rounded, because this will provide an atraumatic proximal end of the probe.

On the other hand, if the proximal tip portion is not the proximal end of the anal probe, then the closed end may be of any shape, because this end will be situated within the retention part.

A closed proximal tip portion may be an advantage because the liquid exiting the proximal tip portion will exit in a diffused manner by forcing the liquid to exit sideways of the probe and then be directed proximally by the retention part. Diffusing of the liquid will prevent any direct spraying of the liquid at the inside of the bowels, which in extreme circumstances may cause trauma to the bowels.

The eyelets may be 1-5 mm wide and 2-6 mm long. In this context the width is transverse to the probe and the length is the longitudinal direction of the probe.

Embodiments relate to the retention part being funnel-shaped. This means that the retention part flares out from a distal portion of the retention part to the proximal end of the retention part, such that a radial extent at the proximal end is larger than a radial extent of the distal portion.

Embodiments relate to the retention part being bowl-shaped. This means that the retention part has a middle port with a radial extent larger than the radial extent of the distal portion and the radial extent of the proximal end. In other words, a circumference of the retention part increases from a first circumference in a distal portion to a second circumference in a middle portion and the decreases again to a third circumference in a proximal end.

Embodiments relate to a radial distance from an outer surface of the proximal tip portion to an inner edge of the proximal end of the retention part being at least 5 mm. This embodiment leaves room for the liquid to leave the proximal tip portion of the stem without being too much interfered or even obstructed by retention part.

In an embodiment, a distal surface of the retention part is impermeable to liquid. By impermeable to liquid is meant that leakage through the retention part is less than 5 ml/min. A leakage in that order, i.e. less than 5 ml/min, will allow the irrigation liquid to stay in the bowels for approximately 5 minutes, which is contemplated to be adequate for the irrigation procedure to be effective.

Embodiments relate to the impermeability being due to the retention part being coated on an outer surface. The coating can be done by dipping or spraying.

Embodiments relate to the impermeability being due to a layer of film-material being added to the retention part on an outer surface thereof. The layer may be vacuum-drawn onto the retention part or thermoformed onto the retention part. Alternatively, the film layer can be manufactured in a specific shape adapted for being put on the retention part.

Embodiments relate to the impermeability being due to a moulded part being added to the retention part on an outer surface thereof. This may be of silicone or TPE and being injection moulded in a shape configured to be put on the retention part.

Embodiments relate to the impermeability being due to the foam having a skin, which is impermeable. The skin may be made during the foaming process, where it is possible to make the outer surface as a closed skin instead of an open-celled structure.

Embodiments relate to the impermeability being due to the foam being a closed cell foam, whereby all of the foam element constituting the retention part is impermeable to liquid.

Embodiments relate to the probe being provided with a film-element configured for keeping the probe in a storage configuration, in which the retention part of the probe is compressed, such that a radial extent of the probe in the storage configuration is smaller than a radial extent of the probe in the use configuration. A smaller diameter may be around 1 cm, such as between 8 mm and 12 mm. The retention part of the probe is able to be compressed because it is made of a soft resilient material. In examples, a retention part of foam is able to be compressed to a small diameter, such as mentioned above.

In embodiments, where the probe comprises foldable arms, these are, in the storage configuration, folded up inside the probe such that the probe can be compressed.

Embodiments relate to the film-element being made of a water-soluble film. Water-soluble films are known from e.g. dishwasher tabs and may be made of Polyvinyl Alcohol (PVA).

If the film-element is made of a water-soluble film, the user does not have to unpack it prior to inserting it. The user just has to insert the probe while it is wrapped in the film, and then the film will dissolve by itself. The process of dissolving the film-element will typically take between 5 and 30 seconds. This process will occur as a result of the liquid being naturally present in the bowel. The dissolving process may be enhanced, if the retention part is made of foam and becomes saturated by the irrigation liquid, because the irrigation liquid will permeate through the retention part to the distal surface of it and assist in dissolving the water-soluble film.

### Detailed Description of the Drawing

Initially, it shall be noted that the figures are schematic illustrations intended only to address the principles and functions of the anal probe described herein and are not to be considered limiting to the scope of the attached claims. Furthermore, the figures and particularly the individually illustrated elements are not necessarily to scale, neither individually nor in relation to each other.

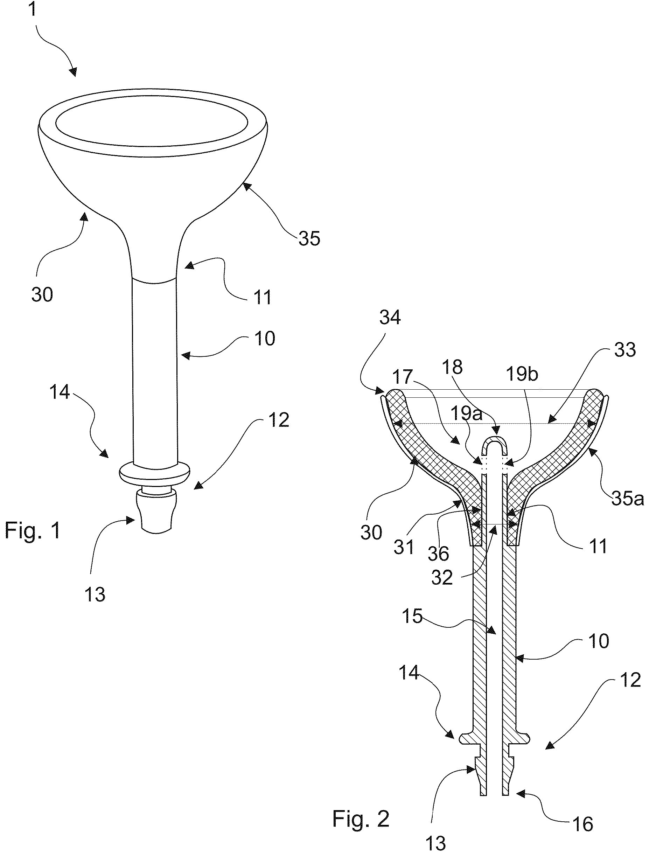

Figures 1 and 2 illustrate an anal probe 1. Figure 1 illustrates a perspective view and figure 2 illustrates a cross-sectional view of the same probe 1. The anal probe consists of a stem 10 and a retention part 30 attached to an attachment portion 11 of the stem 10. In a distal portion 12 of the stem, the stem has a connector 13 configured for attaching it to a tube. In the illustrated embodiment, the connector 13 is illustrated as being of a snap-fit type connector, however, other types of connectors, such as bayonet, friction fit etc. may be contemplated. Furthermore, in the illustrated embodiment, the stem 10 has a flange 14, which functions as a support and holding grip during handling of the anal probe. The retention part 30 is in its distal portion 31 attached to the attachment portion 11 of the stem. The retention part flares out in a proximal direction such that a radial extent 32 of the distal portion of the retention part is smaller than a radial extent 33 of a portion of the retention part, which is proximal to that (see figure 2).

The retention part 30 is here funnel shaped. Towards, the bottom of the funnel, at the distal portion 31, the retention part is attached to the attachment portion 11 of the stem. This is best seen in figure 2. The attachment can be done in a variety of ways, adhering, moulding, snapping on etc. The retention part 30 comprises a central bore 36 at the distal portion. This central bore 36 is configured for being fixedly attached to the stem at the attachment portion 11 of the stem. As can be seen from figure 2, the stem has an internal lumen 15, which extends through the stem from its distal end 16 to a proximal tip portion 17. A proximal tip portion 17 of the stem extends into the flared, funnel-shaped portion of the retention part. The proximal end 18 of the tip portion, and thereby the proximal end of the stem, is closed by rounding it off in a half-spherical manner. Distally of that closed proximal end 18, the tip portion 17 is provided with eyelets 19a, 19b. The proximal end 34 of the retention part extends proximally beyond the proximal end 18 of the stem. This is particularly clear from figure 2. An outer surface 35 (also called a distal surface) of the retention part 30 is impermeable. In this probe, the impermeable surface is due to a thin film-layer 35a attached to the retention part - see figure 2.

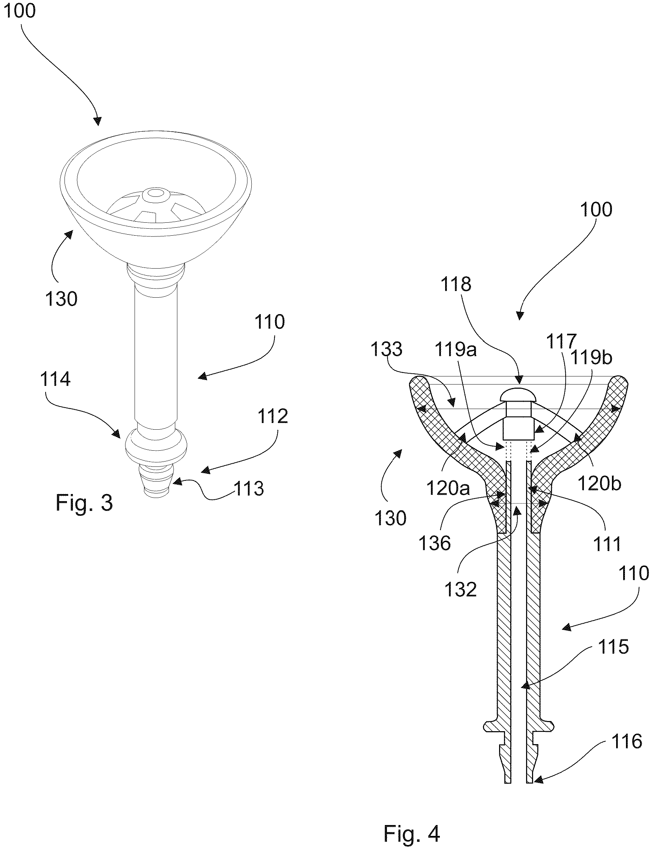

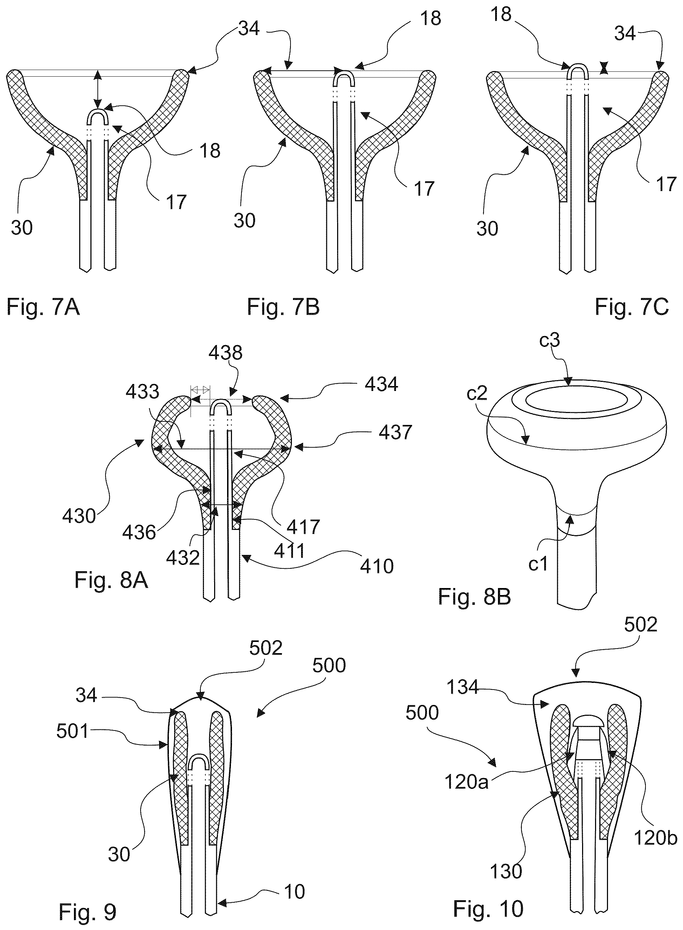

Figures 3 and 4 illustrate another anal probe 100. Figure 3 illustrates a perspective view and figure 4 illustrates a cross-sectional view of the same probe 100. Like with the anal probe of figure 1 and 2, this anal probe consists of a stem 110 and a retention part 130 attached to an attachment portion 111 of the stem 110. This probe also has a connector 113 in a distal portion 112 of the stem and a holding flange 114 positioned proximally of the connector. The retention part 130 flares out in a proximal direction such that a radial extent 132 of the distal portion of the retention part is smaller than a radial extent 133 of a portion of the retention part, which is proximal to that (see figure 4). The attachment or connection between the retention part 130 and the stem 110 can be done as described in relation to figures 1 and 2, and for this purpose, the retention part 130 has a central bore 136 configured for being fixedly attached to the attachment portion 111 of the stem. As can be seen from figure 4, the stem has an internal lumen 115, which extends through the stem from its distal end 116 to a proximal tip portion 117 of the stem. The proximal tip portion 117 extends into the flared, funnel-shaped portion of the retention part, such that the proximal tip portion extends proximally beyond the attachment portion 111. The proximal end 118 of the tip portion, and thereby the proximal end of the stem, is closed by rounding it off in a half-spherical manner. Distally of that closed proximal end 118, the proximal tip portion 117 is provided with eyelets 119a, 119a. Furthermore, in this anal probe, the proximal tip portion 117 is provided with arms 120a, 120b, which can be folded in for compression of the retention part (see figure 10). In figure 4, two arms 120a, 120b can be seen, however three, four, five or six arms may be used. These arms 120a, 120b assists in providing structure for the retention part, in case the retention part is made of a soft material, e.g. a soft foam.

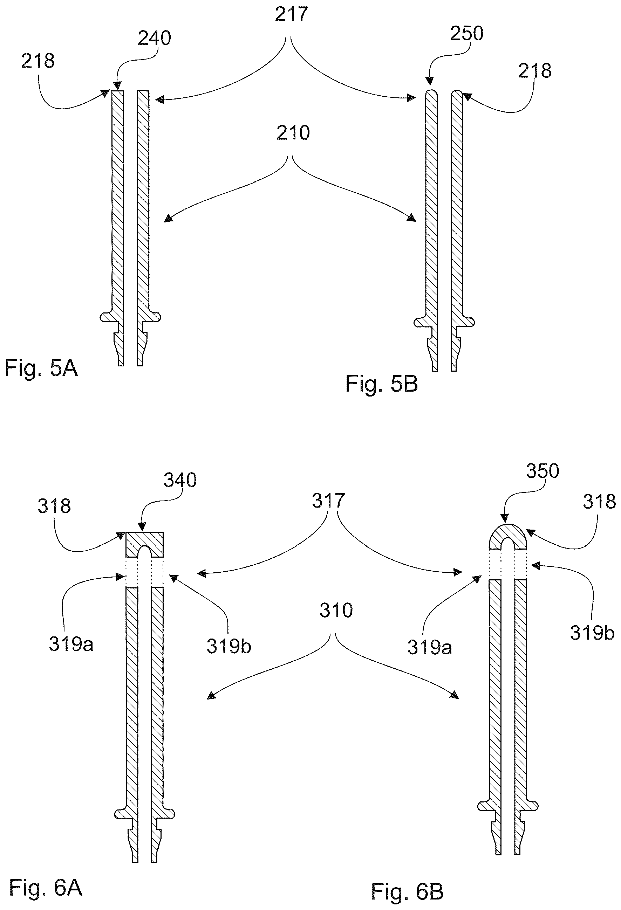

Figures 5A, 5B, 6A, 6B illustrate stems 210, 310, with various proximal tip portions 217, 317. In figures 5A and 5B, the stem 210 is provided with an open-ended tip portion 217, thereby providing an anal probe with such a stem with an open tip. In figure 5A, the tip portion 217 terminates in a proximal end 218 in a flat surface 240. In figure 5B, the tip portion 217 terminates in a proximal end 218 in a rounded surface 250. The rounded surface has the effect of being able to reduce or alleviate trauma, should the proximal end come into contact with the inside of the bowels.

In figures 6A, 6B the stem 310 is provided with a closed ended proximal tip portion 317, thereby providing an anal probe with such a stem with closed tip. The proximal tip portion 317 is provided with eyelets 319a, 319b. In figure 6A, the proximal tip portion 317 terminates in proximal end 318 in a flat surface 340. In figure 6B, the proximal tip portion 317 terminates in a proximal end in a rounded surface 350. The rounded surface has the effect of being able to reduce or alleviate trauma, should the proximal end come into contact with the inside of the bowels.

Figures 7A, 7B, 7C illustrate part of probes similar to the one of figures 1 and 2, and how the proximal tip portion 17 extends into the retention part 30. The distal portion of the stem is missing from these figures, so only a part of the probes is illustrated. All other features are as described in relation to figures 1 and 2 and will not be repeated here.

In figure 7A, the proximal tip portion 17 extends into the retention part such that a proximal end 34 of the retention part extends proximally beyond the proximal end 18 of the proximal tip portion 17. In figure 7B, the proximal tip portion 17 extends into the retention part such that a proximal end 34 of the retention part is radially aligned with the proximal end 18 of the proximal tip portion 17. In figure 7C, the proximal tip portion 17 extends into the retention part such that a proximal end 18 of the tip portion 17 extends proximally beyond the proximal end 34 of the retention part. In other words, in figure 7A, the proximal tip portion is within the retention part, in figure 7B, the proximal tip portion is aligned with the rim of the retention part and in figure 7C, the proximal tip portion extends beyond the rim of the retention part.

Figures 8A, 8B illustrate a part of an anal probe 400. Figure 8A illustrate a cross-sectional view and figure 8B illustrate a perspective view. The distal portion of the stem is missing from these figures - this distal portion may be as indicated in figures 1 to 4.

In the anal probe 400 of this figure, the retention part 430 is not funnel-shaped but rather shaped as a bowl. This means that the retention part still has a radial extent 432 at a distal portion, which is smaller than a radial extent 433 at a position proximally to that, e.g. at a middle portion 437. However, at a proximal end 434 (at the rim of the retention part), the radial extent 438 of the retention part is smaller than the radial extent of the middle portion 437. As can be seen from figure 8B, a circumference c1 at a proximal portion is smaller than a circumference c2 at a middle portion. The circumference c2 at the middle portion is larger than a circumference c3 at a proximal end. Also in this probe, the retention part 430 has a central bore 436 configured for being attached to an attachment portion 111 of the stem.

Figure 9 illustrates part of an anal probe 1 as in figure 1-2 in a storage configuration, where the probe 1 is wrapped in a film-element 500. In particular, the retention part 30 is compressed to be kept in storage configuration, in which the diameter of the retention part is significantly smaller than when it is in a use configuration. As the figure illustrates, the film-element 500 has a sidewall 501 and a top wall 502. The sidewall is attached to the stem 30 at a position below the retention part, i.e. distally of the retention part. The sidewall 501 extends proximally beyond the rim at a proximal end 34 of the retention part and transitions into a top wall 502 covering the entire proximal end 34 of the retention part.

Figure 10 illustrates a part of an anal probe 100 as in figure 3-4 in a storage configuration. As in figure 9, the retention part 130 is wrapped in a film-element and compressed to be kept in a storage configuration. Furthermore, figure 10 illustrates how the foldable arms 120a, 120b are folded up inside the retention part 130.

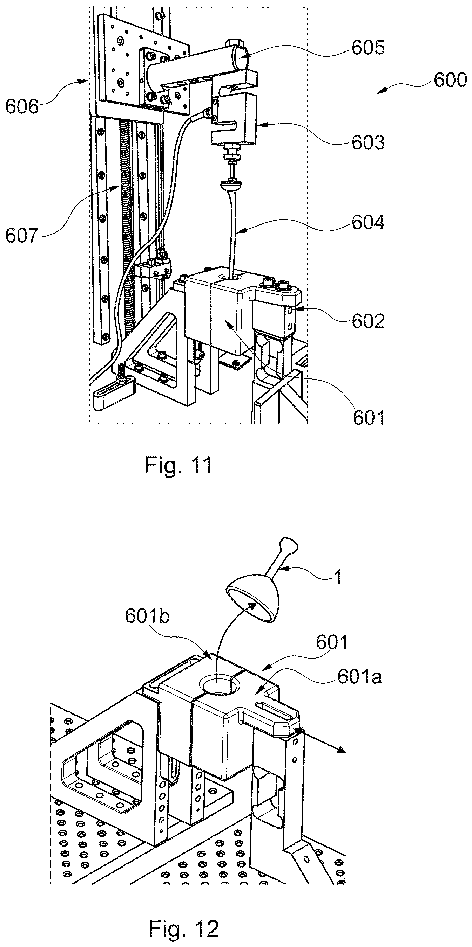

Figure 11 illustrates a test set-up 600 used for testing a pull-out force in vertical and radial direction. Results are shown below under Examples. The test set-up has a cavity 601 for holding the anal probe (see figure 12). Furthermore, two load cells are used, one for tracking the force in radial direction 602 and one for tracking the force in vertical direction 603. The test specimen is pulled by a rod 604 attached to a horizontal bar 605 with the vertical load cell 603 between them. The horizontal bar 605 is part of the Instron testing machine 606 used in this test set-up. The bar 605 can move up on down along with a movable plate 606 mounted on a spindle 607. During testing, the horizontal bar moves 605 upwards and the load cells 602 and 603 reads the resulting force in a radial and vertical direction, respectively.

Figure 12 illustrates how the cavity is divided into two half parts 601a and 601b, where the half-part 601a is movable with respect to the stationary half-part 601b as indicated by the arrow. When the cavity 601 is opened, the anal probe 1 can be inserted into the cavity, which is then closed. During testing, the probe 1 is pulled out of the closed cavity 601 as indicated by the arrow.

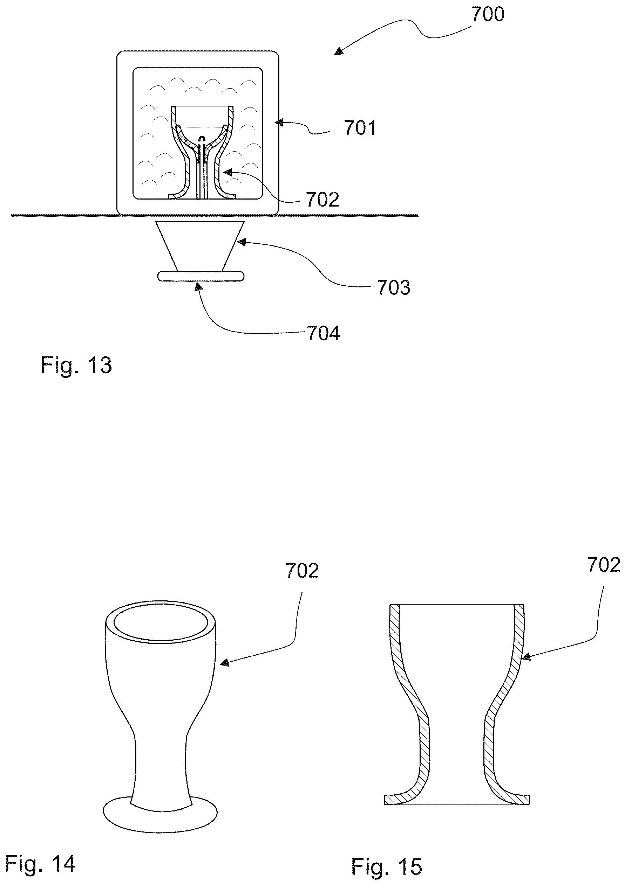

Figures 13 to 15 illustrate a test set-up used for testing drop pressure and leakage on an anal probe. The test set-up in figure 13 shows a test chamber 701 holding a cavity 702, which can be seen in figures 14 and 15. The cavity 702 has a bowl-shape so as to simulate the inside of the bowel. The test chamber 701 is configured for being filled with liquid (as indicated in figure 13) and then be subjected to pressure. During testing, the pressure inside the test chamber 701 may be tracked by a pressure sensor (not shown). A probe 1 is inserted in the cavity 702 during testing. Leakage through the probe is detected by liquid being collected in a container 703 positioned on a scale 704. Furthermore, it is noted at which pressure, the probe 1 falls out of the cavity 702.

Embodiments, and features of the various exemplary embodiments described in this application, may be combined with each other ("mixed and matched"), unless specifically noted otherwise.

### Examples

Four parameters were tested on different types of anal probes. The aim was to determine how to make an anal probe that performed acceptably on these four parameters. The parameters are:
- Vertical force during pull-out
- Radial force during pull-out
- Drop out pressure
- Leakage.

In Table 1 below, the target values for the different parameters are shown.

These target values are based on testing of a known anal plug (Conveen^{®} Anal Plug, marketed by Coloplast A/S) and considerations of factors in relation to use of an anal probe.

**Table 1**

| Parameter | Unit | value |
|---|---|---|
| Vertical force | [N] | 15 N |
| Radial force | [N] | As low as possible |
| Drop out pressure | [mbar] | 150-600 |
| Leakage | [ml/min] | As low as possible (preferably close to zero) |

For the vertical and radial pull-out forces, accepted values are considered to be lower than 15 N. This is based on testing a prior art product, the Conveen^{®} anal plug marketed by Coloplast A/S. The Conveen^{®} anal plug has a vertical pull-out force of 12 N and a radial pull-out force of 10N. The drop out pressure of the Conveen^{®} anal plug is around 600 mbar. The leakage through the anal plug is very high, because it is not constructed to prevent leakage of liquid.

There is a balance between the drop out pressure, which should be as high as possible and the pull-out force, which should be low - in particular in a radial direction. A high pull-out force in a radial direction could potentially lead to trauma in the anal opening during pull-out of the anal probe.

The sealing against leakage has to be close to zero. An acceptable level is considered to be around 5 ml/min. If this is not the case, then the anal probe will not be able to keep the irrigation inside the bowel for the necessary time (around 5 minutes or more).

A lower limit for the drop out pressure is around 150 mbar - the pressure inside the bowel will rarely exceed this value in a normal functioning bowel.

Various anal probes were tested, and the four parameters were noted.

The first two parameters - pull out force and radial force were tested in a test rig as shown in figure 11.

The test rig comprises a bowl-shaped holder, in which the test specimen (e.g. the anal probe) was inserted during testing. The test rig used was a standard tension testing machine, which was connected to two load cells measuring the vertical pull-out force and the radial pull-out force, respectively. The test was done such that the machine-head moved vertically upward by a test speed of 5 mm/s. The tested probes were lubricated prior to testing, so as to simulate the surface of the catheter during insertion and pull-out into the rectum of a user.

Figure 12 illustrates how the anal probe 1 is pulled out of the cavity 601 holding it during testing.

The parameters of leakage and drop-out pressure were tested in a test rig as shown in figure 13. A probe was inserted into the cavity in the test chamber, the test chamber was filled with liquid and put under pressure by a pump (not shown in the figure). The pressure was continuously detected by a pressure sensor (not shown in the figure). The pressure was increased until the anal probe fell out of the cavity or the maximum level of 600 mbar was reached. Leakage was detected by liquid being collected in a container positioned on a scale below the test chamber.

Testing of various probes show that probes with retention parts made of foam with no impermeable outer surface will not perform well with respect to leakage. The leakage through such a probe may be up to 1000 ml/min or more, which is clearly above target. However, if a foam probe is provided with an impermeable layer, e.g. in the form of a thin film, it is possible to reach an acceptable level of leakage - or even as low as close to zero leakage.

With respect to the pull-out force in vertical and radial direction, tests show that it is possible to reach levels of around 5-7 N or even as low as 3 N, for retention parts made of foam with or without foldable arms to support it. The lower values are obtained for probes as in figure 1 without foldable arms.

### Embodiments

1. An anal probe for use in an anal irrigation procedure, the anal probe comprising
   - a stem having a distal end and a proximal tip portion, the stem comprising an internal lumen extending from the distal end to the proximal tip portion
   - a retention part configured for being inserted into the rectum and having a distal portion and a proximal portion, the distal portion having a first radial extent which is smaller than a second radial extent proximally of the distal portion,
   wherein the distal portion of the retention part comprises a central bore, which is fixedly attached, connected to or moulded to the stem at an attachment portion of the stem between the proximal tip portion of the stem and the distal end of the stem.
2. The anal probe as in embodiment 1, wherein the tip is an open tip.
3. The anal probe as in embodiment 1, wherein the tip is a closed tip comprising at least one eyelet, such as two eyelets.
4. The anal probe as in any of the preceding embodiments, wherein the proximal tip portion of the stem extends at least 10 mm proximally beyond the attachment portion.
5. The anal probe as in any of the preceding embodiments, wherein the proximal tip portion of the stem extends proximally beyond a proximal end of the retention part.
6. The anal probe as in any of the preceding embodiments, wherein the proximal end of the retention part extends proximally beyond the proximal tip portion of the stem.
7. The anal probe as in any of the preceding embodiments, wherein the retention part is bowl-shaped.
8. The anal probe as in any of the preceding embodiments, wherein the retention part is conical.
9. The anal probe as in any of the preceding embodiments, wherein a middle portion of the retention part has a radial extent which is larger than a radial extent of the proximal end of the retention part.
10. The anal probe as in any of the preceding embodiments, wherein a radial distance from an outer surface of the tip to an inner edge of the proximal end of the retention part is at least 10 mm.
11. The anal probe as in any of the preceding embodiments, wherein the retention part is made of a soft resilient material.
12. The anal probe as in any of the preceding embodiments, wherein the retention part is made of foam.
13. The anal probe as in any of the preceding embodiments, wherein the retention part is made of silicone.
14. The anal probe as in any of the preceding embodiments, wherein a distal surface of the retention part is impermeable to liquid.
15. The anal probe as in any of the preceding embodiments, wherein a distal surface of the retention part is covered by a layer of film material configured to make the distal surface impermeable to liquid.
16. The anal probe as in any of the preceding embodiments, wherein a distal surface of the retention part is coated with a coating configured to make the distal surface impermeable to liquid.
17. The anal probe as in any of the preceding embodiments, wherein the probe is provided with a film-element configured for keeping the probe in a storage configuration, in which the retention part of the probe is compressed, such that a radial extent of the probe in the storage configuration is smaller than a radial extent of the probe in a use configuration.
18. The anal probe as in any of the preceding embodiments, wherein the film-element configured for keeping the probe in a storage configuration is made of a water-soluble film.

## Claims

1. An anal probe for use in an anal irrigation procedure, the anal probe comprising
- a stem having a distal end and a proximal tip portion, the stem comprising an internal lumen extending from the distal end to the proximal tip portion
- a retention part configured for being inserted into the rectum and having a distal portion and a proximal portion, the distal portion having a first radial extent which is smaller than a second radial extent proximally of the distal portion,
wherein the distal portion of the retention part comprises a central bore, which is fixedly attached, connected to or moulded to the stem at an attachment portion of the stem between the proximal tip portion of the stem and the distal end of the stem, **characterised in that** the retention part is bowl-shaped or conical and wherein the probe is provided with a film-element configured for keeping the probe in a storage configuration, in which the retention part of the probe is compressed, such that a radial extent of the probe in the storage configuration is smaller than a radial extent of the probe in a use configuration, wherein the film-element configured for keeping the probe in a storage configuration is made of a water-soluble film.

2. The anal probe as in claim 1, wherein the tip is an open tip.

3. The anal probe as in claim 1, wherein the tip is a closed tip comprising at least one eyelet, such as two eyelets.

4. The anal probe as in any of the preceding claims, wherein the proximal tip portion of the stem extends at least 10 mm proximally beyond the attachment portion.

5. The anal probe as in any of the preceding claims, wherein the proximal tip portion of the stem extends proximally beyond a proximal end of the retention part.

6. The anal probe as in any of the preceding claims, wherein the proximal end of the retention part extends proximally beyond the proximal tip portion of the stem.

7. The anal probe as in any of the preceding claims, wherein a middle portion of the retention part has a radial extent which is larger than a radial extent of the proximal end of the retention part.

8. The anal probe as in any of the preceding claims, wherein a radial distance from an outer surface of the tip to an inner edge of the proximal end of the retention part is at least 10 mm.

9. The anal probe as in any of the preceding claims, wherein the retention part is made of a soft resilient material.

10. The anal probe as in any of the preceding claims, wherein the retention part is made of foam.

11. The anal probe as in any of the preceding claims, wherein the retention part is made of silicone.

12. The anal probe as in any of the preceding claims, wherein a distal surface of the retention part is impermeable to liquid.

13. The anal probe as in any of the preceding claims, wherein a distal surface of the retention part is covered by a layer of film material configured to make the distal surface impermeable to liquid.

14. The anal probe as in any of the preceding claims, wherein a distal surface of the retention part is coated with a coating configured to make the distal surface impermeable to liquid.
